(19) Europäisches Patentamt
European Patent Office
Office européen des brevets

(11) Publication number : **0 347 199 B1**

# (12) EUROPEAN PATENT SPECIFICATION

(45) Date of publication of patent specification : **09.09.92 Bulletin 92/37**

(51) Int. Cl.$^5$ : **A61K 7/075**

(21) Application number : **89306012.9**

(22) Date of filing : **14.06.89**

## (54) Shampoo composition.

(30) Priority : **16.06.88 GB 8814296**

(43) Date of publication of application : **20.12.89 Bulletin 89/51**

(45) Publication of the grant of the patent : **09.09.92 Bulletin 92/37**

(84) Designated Contracting States : **AT BE CH DE ES FR GB GR IT LI NL SE**

(56) References cited :
**EP-A- 0 023 676**
**EP-A- 0 071 411**
**EP-A- 0 117 135**
**GB-A- 1 440 975**
**US-A- 4 329 334**

(73) Proprietor : **UNILEVER PLC**
**Unilever House Blackfriars P.O. Box 68**
**London EC4P 4BQ (GB)**
(84) **GB**
Proprietor : **UNILEVER N.V.**
**Burgemeester s'Jacobplein 1 P.O. Box 760**
**NL-3000 DK Rotterdam (NL)**
(84) **BE CH DE ES FR GR IT LI NL SE AT**

(72) Inventor : **Murray, Andrew Malcolm**
**15 Moorings Close Parkgate**
**South Wirral Cheshire (GB)**

(74) Representative : **Tonge, Robert James et al**
**UNILEVER PLC Patent Division Colworth House Sharnbrook**
**GB-Bedford MK44 1LQ (GB)**

**EP 0 347 199 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### FIELD OF THE INVENTION

This invention relates to shampoo compositions and more particularly to anti-dandruff shampoos containing an antimicrobial agent which is soluble in an aqueous solution of anionic detergent.

Certain 1-hydroxy-2-pyridone derivatives can be used as anti-dandruff agents. These agents have conventionally been employed in shampoo formulations containing anionic detergents such as lauryl ether sulphates or lauryl sulphates. Substances such as anti-dandruff agents must be retained on washed surfaces in order to produce their intended effect. The present invention is concerned with enhancing the retention of such anti-dandruff agents onto hair and scalp after the shampooing process while preserving the optimum foaming properties of the composition.

### PRIOR ART

GB 1 440 975 (Hoechst) discloses a composition containing 1-hydroxy-2-pyridone or derivatives thereof and surfactants, including sulphosuccinic acid half- and di-esters. There is no indication that any particular anionic surfactant gives foam properties that are superior to other such surfactants which may be included in the composition.

EP 117 135 (Johnson & Johnson) describes a composition which includes anionic surfactant (e.g. monoalkylsulphosuccinate) and an anti-bacterial agent (e.g. 1-hydroxy-2-pyridone or its derivatives). The composition contains also a cationic polymer to enhance retention of the anti-bacterial agent. There is no suggestion that the anionic surfactant would, in the absence of the polymer, enhance retention in this manner.

EP 23 676 (REWO) reports a synergistic effect between an anti-dandruff agent and a sulphosuccinate half ester. However, the anti-dandruff agent employed is a pyridinethione derivative.

### BACKGROUND TO INVENTION

The Applicant has now found that a shampoo having optimum foaming properties, and giving enhanced retention of antimicrobial agent, can be formulated by employing an anti-microbial agent which is soluble in the surfactant together with particular dialkylsulphosuccinate surfactants.

In the case of conventional detergent actives, a reduction in the level of surfactant leads to improved retention of the anti-microbial agent on the skin.

However, there is a corresponding reduction in the foam produced when shampooing. With most detergent actives, high levels of surfactant are needed to provide adequate foam.

Applicants have found surprisingly that by formulating a shampoo to combine a low solubility anti-microbial agent with dialkylsulphosuccinate, there results better retention of the same level of the anti-antimicrobial agent than would occur with an equivalent amount of normal high foaming active such as sodium lauryl ether sulphate 2EO. Alternatively, equivalent retention of the anti-microbial agent may be achieved from a composition containing dialkylsulphosuccinates as from a composition containing conventional high foaming actives with a higher level of the same anti-microbial agent. This means that the amount of expensive anti-microbial agent needed is minimised. In addition, the shampoo foams better than would a conventional surfactant system with equivalent retention.

### DEFINITION OF THE INVENTION

The invention provides an aqueous shampoo composition comprising, in addition to water:

(a) dialkylsulphosuccinate, and

(b) a soluble anti-microbial agent chosen from 1-hydroxy-2-pyridone,

1-chlorophenoxy,1-imidazolyl-2-butanone or derivatives thereof,

wherein the dialkylsulphosuccinate is a sodium or ammonium dialkylsulphosuccinate with alkyl chain lengths of from $C_6$ to $C_9$ or combinations thereof.

### DISCLOSURE OF THE INVENTION

#### (a) Dialkylsulphosuccinate

The composition according to the invention comprises dialkylsulphosuccinate which is an anionic

surfactant. The dialkylsulphosuccinate is present as the sodium or ammonium salt and has alkyl chain lengths of from $C_6$ to $C_9$ or combinations thereof. The alkyl chains may be the same or different, and suitably the average number of carbon atoms in the alkyl chains is from 6.5 to 8.5 carbon atoms per molecule of dialkylsulphosuccinate.

Preferred alkyl chain lengths are combinations of $C_6$ and $C_8$, and the dialkylsulphosuccinate which is especially preferred has alkyl chain lengths $C_6$:$C_8$ in the ratio 40:60.

The dialkylsulphosuccinate is present in the composition in an amount from 1 to 40% by weight, and preferably from 5 to 20% by weight.

(b) Anti-microbial agent

The composition according to the invention also comprises a soluble anti-microbial agent chosen from 1-hydroxy-2-pyridone, 1-chlorophenoxy, 1-imidazolyl-2-butanone or derivatives thereof. The anti-micrbobial agents are soluble in the surfactant system of the shampoo composition.

The preferred 1-hydroxy-2-pyridone derivatives are those of the formula

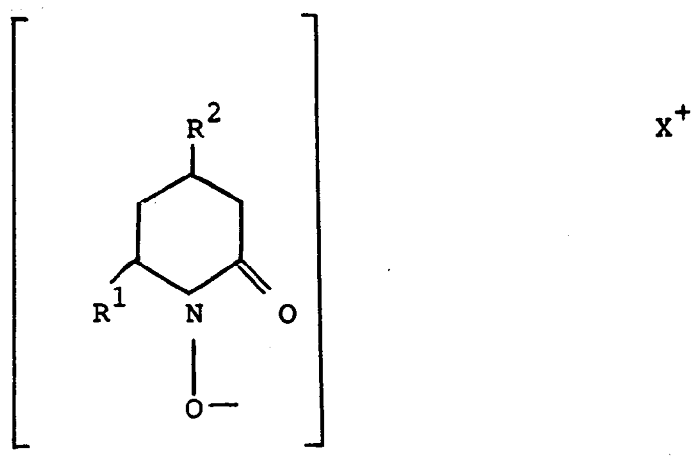

where $R^1$ is:

1-17C alkyl, 2-17C alkenyl, 5-8C cycloalkyl, 7-9C bicycloalkyl, a cycloalkyl-alkyl having 1-4C alkyl, where the cycloalkyl residue may be substituted by a 1-4C alkyl, aryl, aralkyl having 1-4C alkyl, arylalkenyl having 2-4C alkenyl, aryloxyalkyl or aryl mercaptoalkyl having 1-4C alkyl, benzhydryl, phenyl sulphonylalkyl having 1-4C alkyl, furyl or furyl-alkenyl having 2-4C alkenyl, where the aryl residue may be substituted with a 1-4C alkyl, 1-4C alkoxy, $NO_2$, CN, or halogen;

and $R_2$ is:

H or 1-4C alkyl, alkenyl or alkynyl having each 2-4C, halogen, phenyl or a benzyl group;

and X is an organic amine.

The preferred 1-hydroxy-2-pyridone derivative is the compound known as piroctone olamine, whose chemical name is 1-hydroxy-4-methyl-6-(2,4,4-trimethylpentyl)-2-pyridone monoethanolamine salt, and which is sold under the trade name OCTOPIROX by Hoechst AG.

The anti-microbial agent may also be a 1-chlorophenoxy, 1-imidazolyl-2-butanone, or a derivative thereof. The preferred derivative is 1-(4-chlorophenoxy)-1-(1-imidazolyl)-3,3-dimethyl-2-butanone, available commercially from Bayer under the trade name of Climbazole.

The soluble anti-microbial agent is present in the composition in an amount from 0.01 to 10% by weight, and preferably from 0.1 to 2% by weight.

(c) Optional ingredients

The composition of the invention may also comprise other ingredients. Optionally, an additional active may be included so as to improve the retention of the anti-microbial agent on the skin. Examples of such additional actives are alkyl sulphate, alkyl ether sulphate, betaine, amine oxide or mixtures thereof. The additional surfactant is present in the composition in an amount of from 0,1 to 30% or suitably from 1 to 5% by weight.

A hydrotrope, or viscosity and solubility control system, may optionally be included in the composition. An example of a hydrotrope suitable for this purpose is urea. The composition may suitably comprise from 0 to

30% by weight or 1 to 10% by weight of hydrotrope.

The shampoo composition of the invention may also include minor amounts of other ingredients which are commonly employed in shampoos. Examples of such ingredients are foam boosters, viscosity-adjusting agents, opacifiers, pearlescers, perfumes, dyes, colouring agents, conditioning agents, preservatives, thickeners, proteins, polymers and buffering agents.

(d) Water

The composition according to the invention also comprises water in an amount 98.99% by weight or less, forming the balance of the composition.

## THE ASSESSMENT METHOD

In salon tests, the assessment method used was the half head shampooing test. Each test involved 40 panellists. The hair on each panellist's head was made damp with water, then a 3ml dose of one composition to be tested was applied to one half of the head.
Simultaneously, a 3ml dose of a second composition was applied to the other half of the head. The compositions were massaged into the head, without mixing the two, for 1 minute. The hair was rinsed, then dosed with the two shampoo compositions and massaged as before. The two halves of the head were scored by an observer using a Magnitude Estimation method. By this means, either the amount of foam or the anti-dandruff efficacy could be assessed.

## OTHER ASPECTS OF THE INVENTION

Although the invention has so far been described with reference to enhanced retention of an anti-microbial agent from a surfactant system which is a shampoo composition, the principle of retention of a low solubility component is more widely applicable. For example, the retention of sunscreens, perfume or actives other than anti-microbial agents from shampoo or other hair care compositions could be enhanced in this way, in the presence of dialkylsulphosuccinates as defined herein.

## PROCESS

The shampoo composition of the invention is formulated by mixing together the required ingredients in the amounts specified.

## EXAMPLES

The examples below are illustrations of shampoo compositions of this invention. In examples 1, 2, 3, 5 and 6, comparative shampoo formulations are included to illustrate the superior efficacy of the compositions of the invention. The abbreviations used signify the following compounds:

DIAS - dialkylsulphosuccinates of chain lengths $C_6$:$C_8$ in the ratio 40:60
SLES - sodium lauryl ether sulphate (2EO)
CDE - coconut diethanolamide (foam booster)

### Example 1

| Ingredient | % w/w A | % w/w B |
|---|---|---|
| DIAS | 0 | 11.0 |
| SLES | 16.0 | 0 |
| Octopirox | 0.75 | 0.3 |
| Water | to 100% | to 100% |

In vitro studies were conducted which involved treating samples of animal skin, as a model for the human scalp, with composition A or B. It was shown that the Octopirox retention of B was equivalent to that of A. This demonstrates the superior efficacy of Octopirox with dialkylsulphosuccinates by comparison with compositions containing conventional surfactants with higher levels of Octopirox.

Example 2

| Ingredient | % w/w | |
|---|---|---|
| | A | B |
| DIAS | 0 | 9.0 |
| SLES | 12.0 | 0 |
| CDE | 2.0 | 0 |
| Octopirox | 0.3 | 0.3 |
| Water | to 100% | to 100% |

The surfactant system B was shown in vitro to produce an equivalent foam performance to that of A. Thus, a composition containing dialkylsulphosuccinates gives equivalent foam performance to that containing a higher level of conventional surfactant.

Example 3

| Ingredient | % w/w | |
|---|---|---|
| | A | B |
| DIAS | 0 | 11.0 |
| SLES | 16.0 | 0 |
| Octopirox | various | various |
| Water | to 100% | to 100% |

The results of in vitro tests of compositions A and B containing different levels of Octopirox on samples of animal skin are shown in Figure 1. The amount of Octopirox in compositions A and B is plotted against the level of Octopirox retained per $cm^2$ of skin, and it can be seen that, for each level of Octopirox, the retention of Octopirox from B is approximately twice that from A.

Example 4

| Ingredient | % w/w | |
|---|---|---|
| | A | B |
| DIAS | 11.0 | 11.0 |
| SLES | 0 | 1.0 |
| Octopirox | 0.4 | 0.4 |
| Water | to 100% | to 100% |

Comparative testing in vitro showed that the amount of Octopirox retained per $cm^2$ of skin was 1.8µg for A and 2.3µg for B. Thus, the retention of Octopirox from B was superior to that from A, and it can be concluded that retention of DIAS is further enhanced by the addition of SLES to a DIAS base.

Example 5

| Ingredient | % w/w | |
|---|---|---|
| | A | B |
| DIAS | 0 | 11.0 |
| SLES | 16.0 | 1.0 |
| CDE | 3.0 | 0 |
| Octopirox | 0.75 | 0.4 |
| Water | to 100% | to 100% |

Salon tests (half head shampooing tests) showed that the foam produced by B was equivalent to that produced by A.

Example 6

| Ingredient | % w/w | |
|---|---|---|
| | A | B |
| DIAS | 0 | 11.0 |
| SLES (2EO) | 16.0 | 1.0 |
| CDE | 3.0 | 0 |
| Octopirox | 0.75 | 0.4 |
| Water | to 100% | to 100% |

In clinical tests (half head shampooing tests), composition B had an anti-dandruff efficacy equivalent to that of composition A.

Example 7

| Ingredient | % w/w |
|---|---|
| DIAS | 13.0 |
| SLES | 1.0 |
| Octopirox | 1.0 |
| Water | to 100% |

The above composition in accordance with the invention was compared with a commercially-available shampoo containing ammonium lauryl ether sulphate and ammonium lauryl sulphate as surfactant system and 1% zinc pyridinethione as anti-dandruff agent.

In clinical tests (half-head shampooing tests) it was shown that the above composition had an anti-dandruff efficacy significantly better than that of the commercially-available shampoo.

The invention is further illustrated by the following Examples.

Example 8

| Ingredient | % w/w |
|---|---|
| DIAS ($C_6$:$C_8$ in a ratio of 40:60) | 11.0 |
| Opacifier | 5.0 |
| Urea | 1.0 |
| Octopirox | 0.4 |
| Thickener | 2.0 |
| Perfumes, preservatives | q.s |
| Water | to 100% |

Example 9

| Ingredient | % w/w |
|---|---|
| DIAS ($C_6$:$C_8$ in a ratio of 40:60) | 13.0 |
| Lauryl betaine (30% active) | 7.0 |
| Urea | 10.0 |
| Octopirox | 1.0 |
| Thickener | 2.0 |
| Perfumes, preservatives | q.s |
| Water | to 100% |

Example 10

| | % wt |
|---|---|
| Dialkyl suphosuccinate (di-$C_8$) | 12.0 |
| Octopirox | 0.4 |
| Urea | 10.0 |
| Thickener | 2.0 |
| Perfumes/preservatives | q.s. |
| Water | to 100 |

Example 11

| | % wt |
|---|---|
| Dialkylsulphosuccinate ($C_6$:$C_8$ in a ratio of 40:60) | 12.0 |
| Urea | 10.0 |
| Climbazole | 0.1 |
| Thickener | 3.0 |
| Perfume/preservative | q.s. |
| Water | to 100 |

Example 12

| | % wt |
|---|---|
| Dialkylsulphosuccinate ($C_6$:$C_8$ in a ratio of 40:60) | 12.0 |
| Urea | 5.0 |
| Opacifier | 5.0 |
| Climbazole | 0.6 |
| Perfume/preservative | q.s. |
| Water | to 100 |

**Claims**

1. An aqueous shampoo composition comprising, in addition to water:
   (a) dialkylsulphosuccinate, and
   (b) soluble anti-microbial agent chosen from 1-hydroxy-2-pyridone, 1-chlorophenoxy, 1-imidazolyl-2-butanone or derivatives thereof,
   wherein the dialkylsulphosuccinate is a sodium or ammonium dialkylsulphosuccinate with alkyl chain lengths of from $C_6$ to $C_9$ or combinations thereof.

2. An aqueous shampoo composition as claimed in Claim 1 wherein the alkyl chains contain an average of 6.5 to 8.5 carbon atoms per molecule of dialkylsulphosuccinate.

3. A shampoo composition as claimed in Claims 1 or 2, wherein the dialkylsulphosuccinate has alkyl chain lengths $C_6$ and $C_8$ or combinations thereof.

4. A shampoo composition as claimed in any preceding claim wherein the dialkylsulphosuccinate has alkyl chain lengths $C_6$:$C_8$ in the ratio 40:60.

5. A shampoo composition as claimed in any preceding claim wherein the dialkylsulphosuccinate is present in an amount from 1 to 40% by weight.

6. A shampoo composition as claimed in any preceding claim, wherein the dialkylsulphosuccinate is present in an amount from 5 to 20% by weight.

7. A shampoo composition as claimed in any preceding claim, wherein the soluble anti-microbial agent is 1-hydroxy-4-methyl-6-(2,4,4-trimethylpentyl)-2-pyridone monoethanolamine salt.

8. A shampoo composition as claimed in Claims 1 to 6 wherein the soluble anti-microbial agent is 1-(4-chlorophenoxy)-1-(1-imidazolyl)-3,3-dimethyl-2-butanone.

9. A shampoo composition as claimed in any preceding claim, wherein the soluble anti-microbial agent is present in an amount from 0.01 to 10% by weight.

10. A shampoo composition as claimed in any preceding claim, wherein the soluble anti-microbial agent is present in an amount from 0.1 to 2% by weight.

11. A shampoo composition as claimed in any preceding claim further comprising from 0.1 to 30% by weight of an additional surfactant.

12. A shampoo composition as claimed in Claim 11, wherein the additional surfactant is chosen from an alkyl sulphate, an alkyl ether sulphate, a betaine, or an amine oxide, or mixtures thereof.

13. A shampoo composition as claimed in Claim 11 or Claim 12, wherein the additional surfactant is present in an amount from 1 to 5% by weight.

14. A shampoo composition as claimed in any preceding claim, further comprising from 0 to 30% by weight of a hydrotrope.

15. A shampoo composition as claimed in Claim 14, wherein the hydrotrope is urea.

16. A shampoo composition as claimed in Claim 14 or Claim 15, wherein the hydrotrope is present in an amount from 1 to 10% by weight.

## Patentansprüche

1. Wässriges Shampoo-Präparat, das zusätzlich zu Wasser umfaßt:
   (a) Dialkylsulphosuccinat, und
   (b) lösliches antimikrobielles Mittel, ausgewählt aus 1-Hydroxy-2-pyridon, 1-Chlorphenoxy,1-imidazolyl-2-butanon oder Derivate davon,

   wobei das Dialkylsulphosuccinat ein Natrium- oder AmmoniumDialkylsulphosuccinat mit Alkylkettenlängen vom $C_6$ bis $C_9$ oder Kombinationen davon ist.

2. Wässriges Shampoo-Präparat nach Anspruch 1, worin die Alkylketten durchschnittlich 6,5 bis 8,5 Kohlenstoffatome pro Molekül Dialkylsulphosuccinat enthalten.

3. Shampoo-Präparat nach Anspruch 1 oder 2, worin das Dialkylsulphosuccinat Alkylkettenlängen von $C_6$ bis $C_8$ oder Kombinationen davon hat.

4. Shampoo-Präparat nach einem der vorhergehenden Ansprüche, worin das Dialkylsulphosuccinat Alkylkettenlängen $C_6$:$C_8$ im Verhältnis 40:60 hat.

5. Shampoo-Präparat nach einem der vorhergehenden Ansprüche, worin das Dialkylsulphosuccinat in einer Menge von 1 bis 40 Gew.-% vorhanden ist.

6. Shampoo-Präparat nach einem der vorhergehenden Ansprüche, worin das Dialkylsulphosuccinat in einer Menge von 5 bis 20 Gew.-% vorhanden ist.

7. Shampoo-Präparat nach einem der vorhergehenden Ansprüche, worin das lösliche antimikrobielle Mittel 1-Hydroxy-4-methyl-6-(2,4,4-trimethylpentyl)-2-pyridon-monoethanolaminsalz ist.

8. Shampoo-Präparat nach Anspruch 1 bis 6, worin das lösliche antimikrobielle Mittel 1-(4-Chlorphenoxy)-1-(1-imidazolyl)-3,3-dimethyl-2-butanon ist.

9. Shampoo-Präparat nach einem der vorhergehenden Ansprüche, worin das lösliche antimikrobielle Mittel in einer Menge von 0,01 bis 10 Gew.-% vorhanden ist.

10. Shampoo-Präparat nach einem der vorhergehenden Ansprüche, worin das lösliche antimikrobielle Mittel in einer Menge von 0,1 bis 2 Gew.-% vorhanden ist.

11. Shampoo-Präparat nach einem der vorhergehenden Ansprüche, das weiterhin 0,1 bis 30 Gew.-% eines zusätzlichen Tensids umfaßt.

12. Shampoo-Präparat nach Anspruch 11, worin das zusätzliche Tensid ausgewählt ist aus einem Alkylsulfat, einem Alkylethersulfat, einem Betain oder einem Aminoxid oder Gemischen davon.

13. Shampoo-Präparat nach Anspruch 11 oder 12, worin das zusätzliche Tensid in einer Menge von 1 bis 5 Gew.-% vorhanden ist.

14. Shampoo-Präparat nach einem der vorhergehenden Ansprüche, weiterhin 0 bis 30 Gew.-% eines Lösungsvermittlers umfassend.

15. Shampoo-Präparat nach Anspruch 14, worin der Lösungsvermittler Harnstoff ist.

16. Shampoo-Präparat nach Anspruch 14 oder 15, worin der Lösungsvermittler in einer Menge von 1 bis 10 Gew.-% vorhanden ist.

## Revendications

1. Composition aqueuse de shampooing, qui comprend, outre l'eau :
   (a) un dialkylsulfosuccinate, et
   (b) un agent antimicrobien soluble choisi parmi : 1-hydroxy-2-pyridone, 1-chlorophénoxy, 1-imidazolyl-2-butanone ou leurs dérivés,
   dans laquelle le dialkylsulfosuccinate est un dialkylsulfosuccinate de sodium ou d'ammonium ayant une chaîne alkyle d'une longueur de $C_6$ à $C_9$ ou des combinaisons de ceux-ci.

2. Composition aqueuse de shampooing selon la revendication 1, dans laquelle les chaînes alkyle contiennent une moyenne de 6,5 à 8,5 atomes de carbone par molécule de dialkylsulfosuccinate.

3. Composition de shampooing selon la revendication 1 ou 2, dans laquelle le dialkylsulfosuccinate présente des longueurs de chaînes alkyle de $C_6$ et $C_8$ ou des combinaisons de celles-ci.

4. Composition de shampooing selon l'une quelconque des revendications précédentes, dans laquelle le dialkylsulfosuccinate présente des longueurs de chaînes alkyle $C_6$:$C_8$ dans un rapport de 40:60.

5. Composition de shampooing selon l'une quelconque des revendications précédentes, dans laquelle le dialkylsulfosuccinate est présent à raison de 1 à 40% en poids.

6. Composition de shampooing selon l'une quelconque des revendications précédentes, dans laquelle le dialkylsulfosuccinate est présent à raison de 5 à 20% en poids.

7. Composition de shampooing selon l'une quelconque des revendications précédentes, dans laquelle l'agent antimicrobien soluble est un sel de monoéthanolamine de 1-hydroxy-4-méthyl-6-(2,4,4-triméthylpentyl)-2-pyridone.

8. Composition de shampooing selon les revendications 1 à 6, dans laquelle l'agent antimicrobien soluble est la 1-(4-chlorophénoxy)-1-(1-imidazolyl)-3,3-diméthyl-2-butanone.

9. Composition de shampooing selon l'une quelconque des revendications précédentes dans laquelle l'agent antimicrobien soluble est présent à raison de 0,01 à 10% en poids.

**10.** Composition de shampooing selon l'une quelconque des revendications précédentes dans laquelle l'agent antimicrobien soluble est présent à raison de 0,1 à 2% en poids.

**11.** Composition de shampooing selon l'une quelconque des revendications précédentes, qui comprend en outre de 0,1 à 30% en poids d'un tensio-actif supplémentaire.

**12.** Composition de shampooing selon la revendication 11, dans laquelle le tensio-actif supplémentaire est un alkylsulfate, un alkyléthersulfate, une bétaïne, un oxyde d'amine ou un mélange de ceux-ci.

**13.** Composition de shampooing selon la revendication 11 ou 12, dans laquelle le tensio-actif supplémentaire est présent à raison de 1 à 5% en poids.

**14.** Composition de shampooing selon l'une quelconque des revendications précédentes, qui comprend en outre de 0 à 30% en poids d'un hydrotrope.

**15.** Composition de shampooing selon la revendication 14, dans laquelle l'hydrotrope est l'urée.

**16.** Composition de shampooing selon la revendication 14 ou 15, dans laquelle l'hydrotrope est présent à raison de 1 à 10% en poids.

Fig. 1.

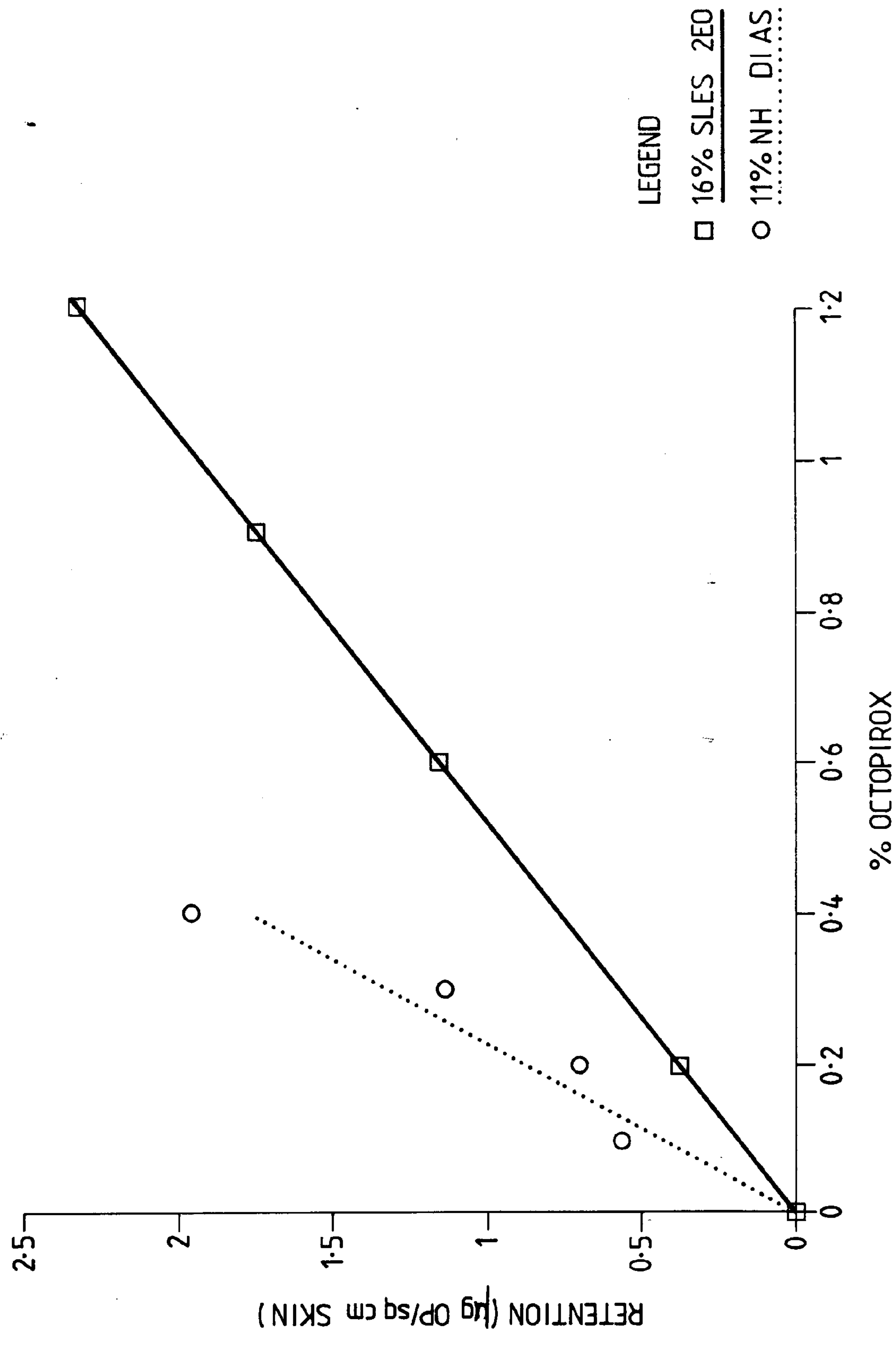
RETENTION (µg OP/sq cm SKIN)
0
0·5
1
1·5
2
2·5
% OCTOPIROX
0
0·2
0·4
0·6
0·8
1
1·2
LEGEND
16% SLES 2EO
11% NH DI AS